# EUROPEAN PATENT SPECIFICATION

(11) **EP 3 932 341 B1**
(45) Date of publication and mention of the grant of the patent: **10.01.2024**
(21) Application number: 21181934.7
(22) Date of filing: 28.06.2021
(51) Int. Cl.: A61B 17/22, A61B 17/221, A61B 17/3207, A61B 17/00, A61M 25/10, A61F 2/958, A61M 25/00, A61F 2/90

(54) **INTRAVASCULAR PLAQUE ISOLATION, DESTABILIZATION, AND ASPIRATION**
ISOLATION, DESTABILISIERUNG UND ASPIRATION VON INTRAVASKULÄRER PLAQUE
ISOLATION, DÉSTABILISATION ET ASPIRATION DE LA PLAQUE INTRAVASCULAIRE

(30) Priority: 29.06.2020 US 202016914612
(43) Date of publication of application: 05.01.2022
(73) Proprietor: Neuravi Limited, Galway H91 K5YD (IE)
(72) Inventor: KEATING, Karl, Galway, H91 K5YD (IE); KELLY, Ronald, Galway, H91 K5YD (IE)
(74) Representative: Carpmaels & Ransford LLP

(56) References cited:
- US-A1- 2018 280 005
- US-B1- 6 319 242
- US-B1- 6 485 500
- US-B2- 6 652 548
- US-B2- 9 408 626

## Description

### Field of Invention

The present invention generally relates to medical devices, and more particularly, to a system for intravascular plaque isolation, destabilization, aspiration, and removal.

### Background

Atherosclerosis results from lesions which narrow and reduce the space in the lumen of vessels in the vasculature. Such lesions are usually composed of plaque, which can be fat, cholesterol, calcium, or other components of the blood. Severe occlusion or closure can impede the flow of oxygenated blood to different organs and parts of the body and result in other cardiovascular disorders such as heart attack or stroke. Narrowing of vessels, or stenosis, increases the risk that clots, and other emboli can lodge at such locations, especially in the neurovascular where vessel diameters are already small. Intracranial atherosclerosis disease (ICAD) is the narrowing of those arteries and vessels supplying blood to the brain and represents the most common proximate mechanism of ischemic stroke.

These clogged blood vessels can lead to blockages that can cause strokes, or, for example, when these blockages break free from the wall of the blood vessel, they can cause a heart attack. These blockages can be treated with medications, stents, surgeries (e.g., bypass surgery), and/or other treatments including an angioplasty. Each of these treatments for blocked blood vessels have certain drawbacks. For example, medication(s) can have various side effects, a patient may be allergic to a stent and/or develop an infection from the stent, and surgeries can result in complications and may only temporarily remedy the issue.

Therefore, there is a need for improved methods, devices, and systems for isolating, destabilizing, aspirating, and removing plaque within blood vessels.

US 6652548 B2 relates to devices, kits, and methods to remove clot material from the vasculature and other body lumens. Expansible baskets are used as cooperating radially expansible shearing members. Helically oriented struts of each basket may wind in a uniform circumferential direction. The struts can be independently flexible, allowing the shearing members to flex axially together.

US 6485500 B1 relates to an emboli protection system that provides one or more inflatable blocking balloons for isolation of a section of a blood vessel to prevent migration of emboli from the section during an interventional procedure, and fluid infusion and evacuation ports for flushing emboli from the isolated section. There is described a distal blocking balloon catheter, over which an interventional device can be introduced, and a proximal blocking balloon catheter to be introduced over the interventional device for isolating a portion of a blood vessel to be treated.

US 9408626 B2 relates to a clot removal system that includes first and second barriers that each have an expanded configuration forming an occlusion within a vessel and a collapsed configuration. At least one deployment device distally deploys the first barrier relative to a clot, and proximally deploys the second barrier relative to the clot. The at least one deployment device also delivers a thrombolytic agent into an isolated segment defined at least in part by the first and second barriers in the expanded configurations, and positions an impeller within the isolated segment.

US 6319242 B1 relates to an apparatus and methods for removing stenotic material from within previously stented regions of a patient's vasculature. The apparatus includes a catheter system having a stenotic material removal mechanism mounted on a distal portion of an elongated inner catheter.

### Summary

It is an object of the present invention to provide a system to meet the above-stated needs. Generally, it is an object of the present invention to provide a system for extracting plaque from a vasculature to meet the above-stated needs. The invention is defined in claim 1. Embodiments of the invention are defined in the dependent claims. The surgical methods disclosed do not form part of the claimed invention.

### Brief Description of the Drawings

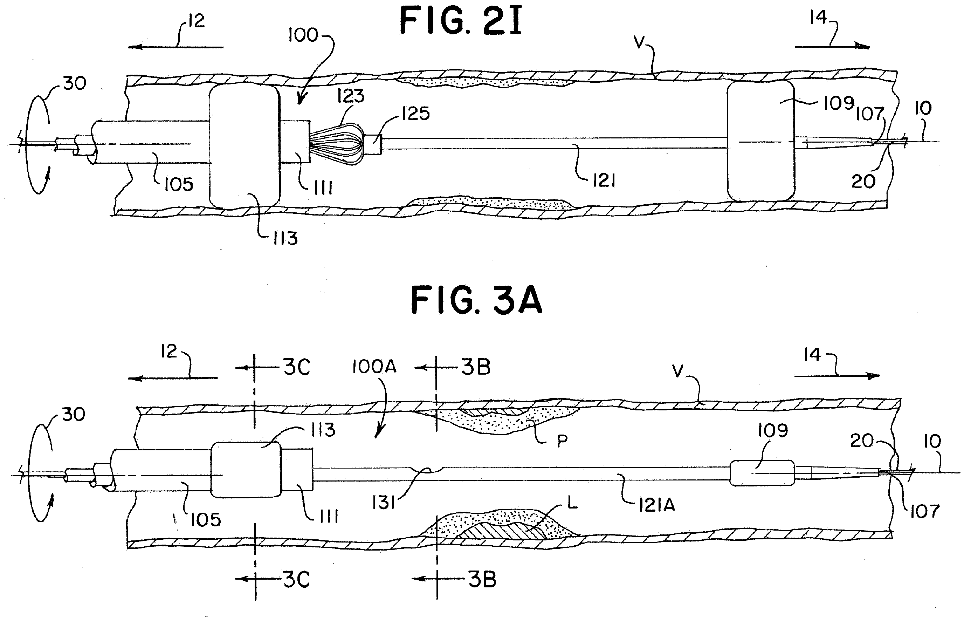

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the disclosed devices, by way of example only, not by way of limitation. A system according to the invention is disclosed in Fig. 3A-3E.
Figure 1 is an illustration of an example intravascular system including an expandable frame for displacing plaque according to aspects of the present disclosure;
Figures 2A-2I illustrate an exemplary sequence of using the intravascular system illustrated in Figure 1 in a plaque displacement method according to aspects of the present disclosure;
Figures 3A through 3E are illustrations of an example intravascular treatment system according to an aspect of the present invention, including an incision device, and another example plaque displacement method according to aspects of the present disclosure;
Figure 4A is an illustration of yet an example intravascular system including agitation veins for displacing plaque according to aspects of the present disclosure;
Figure 4B is an illustration of a cross section of the intravascular system illustrated in Figure 4A;
Figure 5 is an illustration of yet an intravascular system including a balloon expandable structure for displacing plaque according to aspects of the present disclosure;
Figure 6 is an illustration of a thrombectomy device and method step for using the thrombectomy device in conjunction with any of the example intravascular systems according to aspects of the present disclosure;
Figures 7A and 7B are illustrations of a stent and method steps for using the stent with any of the example intravascular systems according to aspects of the present disclosure;
Figure 8 is an example flowchart of a method for displacing and aspirating intravascular plaque according to aspects of the present disclosure; and
Figure 9 is an example flowchart of a method for displacing, aspirating, and flushing intravascular plaque according to aspects of the present disclosure.

### Detailed Description

Some examples presented herein can be used to aid in the displacement and removal of plaque within a vasculature. Some examples presented herein can be used to isolate a lesion during plaque displacement such that plaque located on a blood vessel wall can be displaced and removed from the vasculature while inhibit dislodged plaque fragments from migrating from the treatment site. To meet some or all of these needs, example systems can include an inner tube having a distal occlusion element that can be expanded in the distal direction in relation to the treatment site, a balloon guide catheter having a balloon thereon that can be expanded in the proximal direction in relation to the treatment site and a lumen through which the inner tube can traverse, and a plaque displacement apparatus that can mechanically displace plaque at the treatment site while the distal occlusion element and the proximal balloon are deployed.

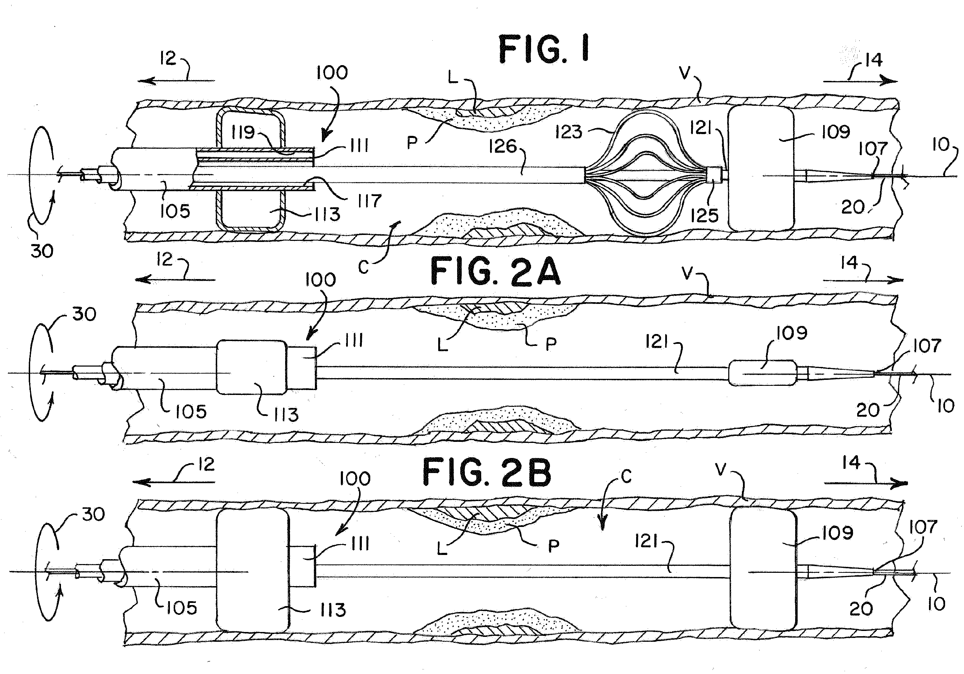

Figure 1 is an illustration of an intravascular system 100 for displacing plaque (P). As shown, the intravascular system 100 can include a guide wire 20, a balloon guide catheter (BGC) 105, an inner tube 121, and a plaque displacement apparatus 123. The BGC 105 can include a BGC distal end 111 and a proximal balloon 113 positioned approximate the BGC distal end 111. The balloon guide catheter 105 can further include the device delivery lumen 117 that extends through the balloon guide catheter 105 and that can be sized to allow the inner tube 121 to slide therethrough and sized to contain the plaque displacement apparatus 126 as the system 100 is delivered intravascularly to the lesion L.

The inner tube 121 can include the tube distal end 107 and the distal occlusion element 109, which can be positioned approximate the tube distal end 107. The distal occlusion element 109 can be collapsible to be contained within the device delivery lumen 117 and expandable to appose vasculature when moved distally out of the device delivery lumen 117.

When the system 100 is deployed as illustrated in Figure 1, the plaque displacement apparatus 123 can move the longitudinal 10 and/or a rotational direction 30 in relation to the balloon guide catheter 105. The system 100 can include a positioning tube 126 attached to the plaque displacement apparatus 123 that can be manipulated to translate the plaque displacement apparatus 123 distally and proximally. In some examples, the positioning tube 126 can further rotate in a rotational direction 30 about the longitudinal axis 10 to cause the plaque displacement apparatus 123 to rotate. The tube 126 can be coaxial with the inner tube 121 and slidably translatable over the inner tube 121. The system 100 can include a collar 125 attached to the plaque displacement apparatus 123 that can slide freely over the inner tube 121. Position of the collar 125 can depend on the position of the tube 126 and the shape of the plaque displacement apparatus 123.

Figures 2A-2I illustrates the intravascular system 100 for displacing plaque at various stages of a treatment. The balloon guide catheter 105 can be used to insert the intravascular system 100 within the vasculature. In some treatments, the balloon guide catheter 105 can be inserted into the femoral artery to perform certain methods disclosed herein that can be performed with the intravascular system 100, 100A, 100B, and/or 100C. Using the guide wire 20, the intravascular system 100 can be threaded in the arterial system until a vasculature lesion L is reached. Once the lesion L is reached, the distal occlusion element 109 can be positioned in a distal direction 14 in relation to the vasculature lesion L. The proximal balloon 113 can be positioned a proximal direction 12 in relation to the intravascular lesion L.

Figure 2A is indicative of the intravascular system 100 after insertion into the vasculature and prior to inflation of the proximal balloon 113 and expansion of the distal occlusion element 109. The distal occlusion element 109 can be inflatable, self-expandable, mechanically expandable, or otherwise expandable as understood by a person of ordinary skill in the art according to the teachings of the present disclosure. The system 100 can include additional components and structures not illustrated herein to facilitate expansion of the distal occlusion element 109. Such components and structures can include, but are not limited to, a sheath which can restrict the distal occlusion element 109 and be retracted proximally to allow the distal occlusion element 109 to expand, an inflation lumen to inflate a balloon of the distal occlusion element 109, or other such structure. Similarly, a self-expandable, mechanically expandable, or other expandable component can be used in place of the proximal balloon 113 as understood by a person of ordinary skill in the art according to the teachings of the present disclosure.

Figure 2B illustrates the intravascular system 100 after inflation of the proximal balloon 113 and expansion of the distal occlusion element 109. At this point, due to the inflation of the proximal balloon 113 and the distal occlusion element 109, the cavity C can be isolated. In some examples, both the proximal balloon 113 and the distal occlusion element 109 can respectively form fluid impermeable seals within the blood vessel V effective to restrict blood flow within the cavity C.

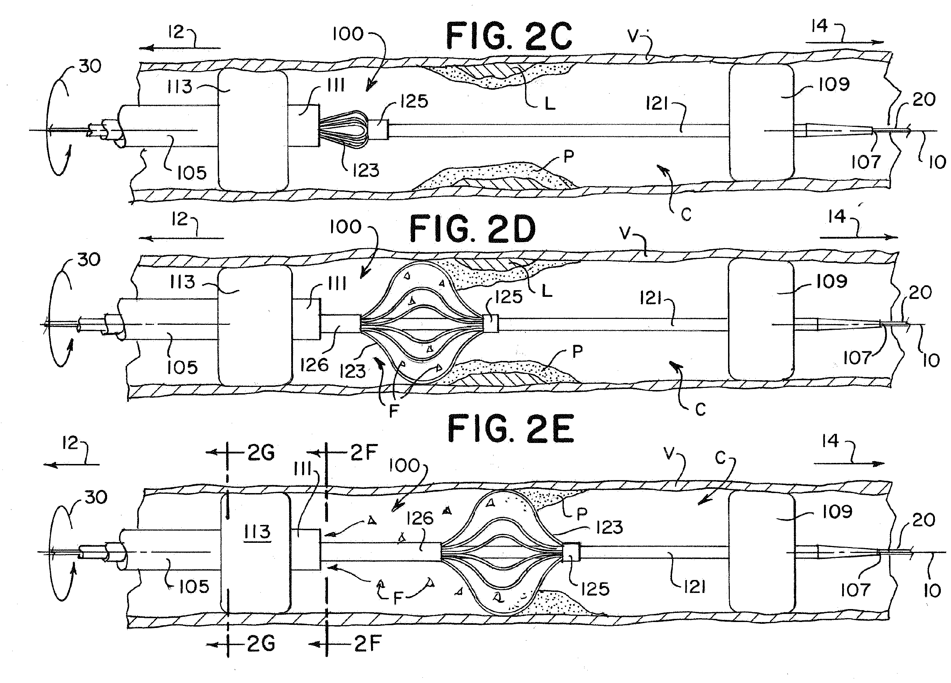

Figure 2C shows the intravascular system 100 with the plaque displacement apparatus 123 partially exposed from the device delivery lumen 117 as it begins moving in the distal direction 14 to exit the balloon guide catheter 105. The plaque displacement apparatus 123 can be inhibited from expanding when positioned in the device delivery lumen 117 and expand as it exits the device delivery lumen 117. The collar 125 can slide freely over the inner tube 121 as the plaque displacement apparatus 123 expands.

Figure 2D shows the adjustable plaque displacement apparatus 123 can increase in circumference as it moves in the longitudinal direction 10 from a proximal position within the device delivery lumen 117 and to a distal position distal to the distal end 111 of the BGC 105. Also shown in Figure 2D, when expanded in circumference, the plaque displacement apparatus 123 can contact the plaque P within the walls of the blood vessel, such that portions (fragments) F of plaque become displaced.

Figure 2E shows the portions F of plaque after becoming displaced. The portions F of plaque can be aspirated into the BGC 105. Preferably, the device delivery lumen 117 and delivery tube 126 are sized and/or otherwise configured to allow portions F of dislodged plaque to be aspirated into the device delivery lumen 117 as the plaque displacement apparatus 123 is moved against the plaque P at the lesion L. Alternatively, the system 100 can include a lumen not illustrated to aspirate the dislodged portions F. For instance, the BGC 105 can include an additional lumen, the inner tube 121 can include an aspiration lumen, and/or the delivery tube 126 can include an aspiration lumen.

In some treatments, fluid can be injected into the cavity C through the flush lumen 119 during aspiration. In examples where both the proximal balloon 113 and the distal occlusion element 109 are effective to arrest blood flow through the vessel V, injective fluid into the cavity C through the flush lumen 119 can reduce the likelihood that vessel V collapses in the vicinity of the cavity C due to negative pressure created by suction from aspiration. Also, fluid (e.g., saline, an anti-thrombogenic drug, a plaque sealant, etc.) can be injected into the cavity through the flush lumen 119. In some examples, this can occur simultaneously with the aspiration of the portions of plaque.

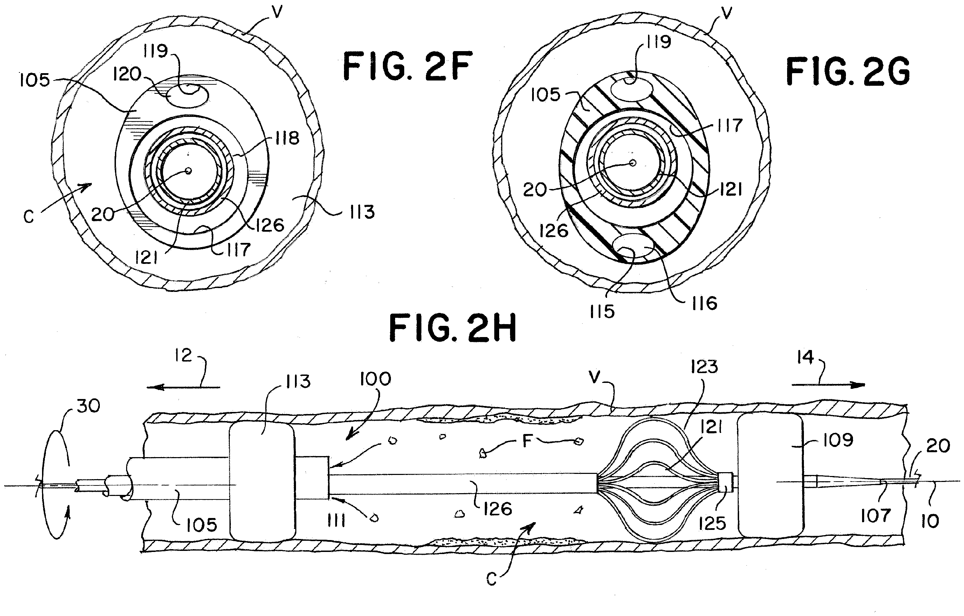

Figure 2F depicts a cross-sectional view of the intravascular system 100 at a position in the distal direction in relation to the distal end 111 of the BGC 105 and looking in the proximal direction 12 as indicated in Figure 2E. At the outermost portion of Figure 2F is the vasculature V with the inflated proximal balloon 113 inflated to appose walls of the blood vessel V. Figure 2F displays the balloon guide catheter 105 at its distal end 111, a cross section of the delivery tube 126 positioned within the device delivery lumen 117 of the BGC 105, a cross sectional view of the inner tube 121 positioned with the delivery tube 126, and a cross sectional view of the guide wire 20 positioned within the inner tube 121.

In this view, the opening 118 of the device delivery lumen 117 is visible. The delivery tube 126, inner tube 121, and guide wire 20 are slidably translatable to enter and exit the lumen 117 via the opening 118.

In this view, an opening 120 of the flush lumen 119 is visible at the distal end 111 of the BGC 105.

Figure 2G depicts a cross-sectional view of the balloon guide catheter 105 as a cross section through the proximal balloon 113 and looking in the proximal direction 12 as indicated in Figure 2E. From this vantage point, an opening 116 to an inflation lumen 115 through the BGC 105 is illustrated. The inflation lumen 115 is configured to provide fluid to the proximal balloon 113 to inflate and deflate the proximal balloon 113.

Figures 2H illustrates the intravascular system 100 with the plaque displacement apparatus extended in the longitudinal direction 10 proximate the tube distal end 107 and the intravascular system 100. The plaque displacement apparatus 123 can be moved in the distal direction 14 and in the proximal direction 12 in relation to the proximal balloon 113 and in relation to the distal occlusion element 109. As shown, the plaque displacement apparatus 123 has longitudinally traversed the plaque along the walls of the vessel V. Traversal of plaque displacement apparatus 123 across the lesion L can be effective to dislodge some or all of the plaque P. In some examples, the plaque displacement apparatus 123 can move rotationally in the rotational direction 30 to agitate the plaque P. In some examples the plaque displacement apparatus 123 can include a structure or component to cause the plaque displacement apparatus to repeatedly expand and contract to make contact and/or puncture the plaque along the blood vessel wall, such that portions of the plaque are freed.

Figure 2I depicts the plaque displacement apparatus 123 can retract in the proximal direction 12 toward the BGC 105. The plaque displacement apparatus 123 can contract to be contained by the device delivery lumen 117 as it is retracted in the proximal direction.

Figure 3A illustrates another example intravascular system 100A for displacing plaque. Similar to the intravascular system 100 illustrated in Figures 1 and 2A through 2I, the intravascular system 100A can include the guide wire 20, the balloon guide catheter 105 with the proximal balloon 113 thereon and an inner tube 121A having the distal occlusion element 109 thereon. The inner tube 121A illustrated in Figure 3A differs from the inner tube 121 illustrated in Figures 1 and 2A through 2I in that the inner tube 121A includes an opening 131 sized to allow a plaque displacement element to pass therethrough. The system 100A can be delivered to the position illustrated in Figure 3A by similar methods as the system 100 illustrated in Figures 1 and 2A through 2I, particularly by methods described in relation to Figure 2A.

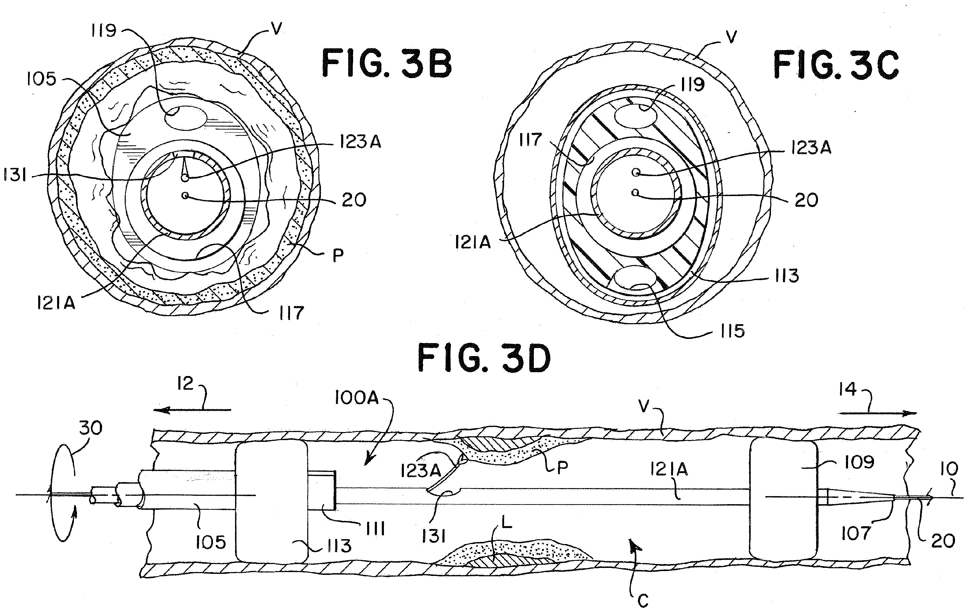

Figure 3B shows a cross-sectional view of the inner tube 121A of the inflated intravascular system 100A at a position which passes through the opening 131 on the inner tube 121A and looking in the proximal direction 12 as indicated in Figure 3A. The system 100A can include an incision tool 123A positioned within the inner tube 121A and movable to exit the opening 131 in the inner tube 121A. Plaque P is illustrated circumferentially attached to walls of the vessel V.

Figure 3C shows a cross-sectional view of the BGC 105 through the proximal balloon 113 and looking in the proximal direction as indicated in Figure 3A. The proximal balloon 113 is deflated. The incision tool 123A is illustrated in cross section. The incision device can include an elongated structure that is movable in the proximal and distal direction in relation to the inner tube 121A.

Figure 3D shows the proximal balloon 113 and distal occlusion element 109 expanded and the incision tool 123A moved to engage the plaque P. The incision tool 123A can be used to puncture and/or cut the lesion L. In some examples, the inner tube 121A can include a rotating joint positioned near the distal occlusion element 109 and/or between the opening 131 and the distal occlusion element 109 so that a proximal portion of the inner tube including the opening 131 can be rotated in the rotational direction 30 as indicated in Figure 3D. The incision tool 123A can be moved against the plaque P, in the rotational direction, as the opening 131 is rotated.

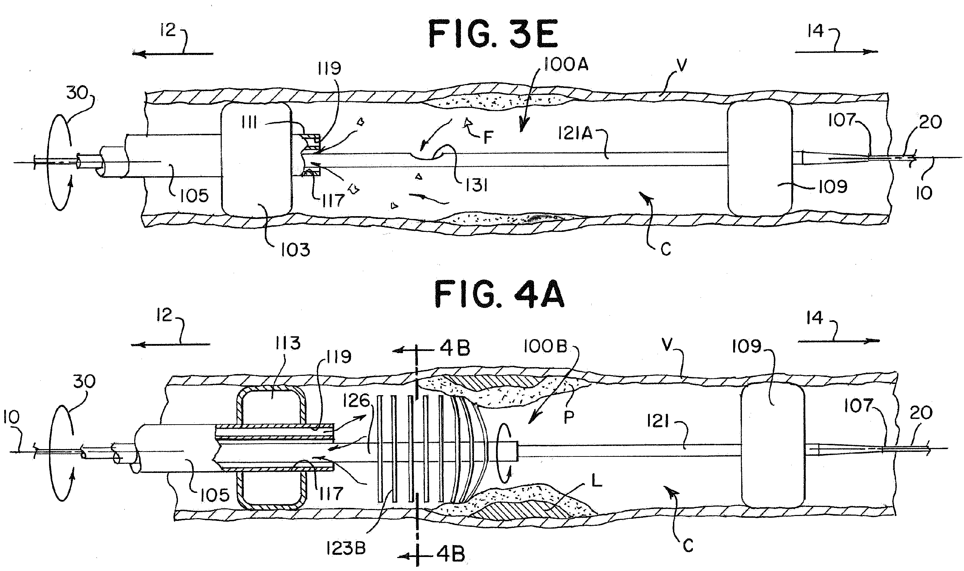

Figure 3E shows aspiration of cavity C through the BGC 105 and/or inner tube 121A. During aspiration, fluid can be provided through the flush lumen. In some treatments, fluid such as saline solution or a drug can be flowed into the cavity C through the flush lumen 119 while aspirating. Rate of aspiration and fluid flow can be regulated to control pressure within the cavity C.

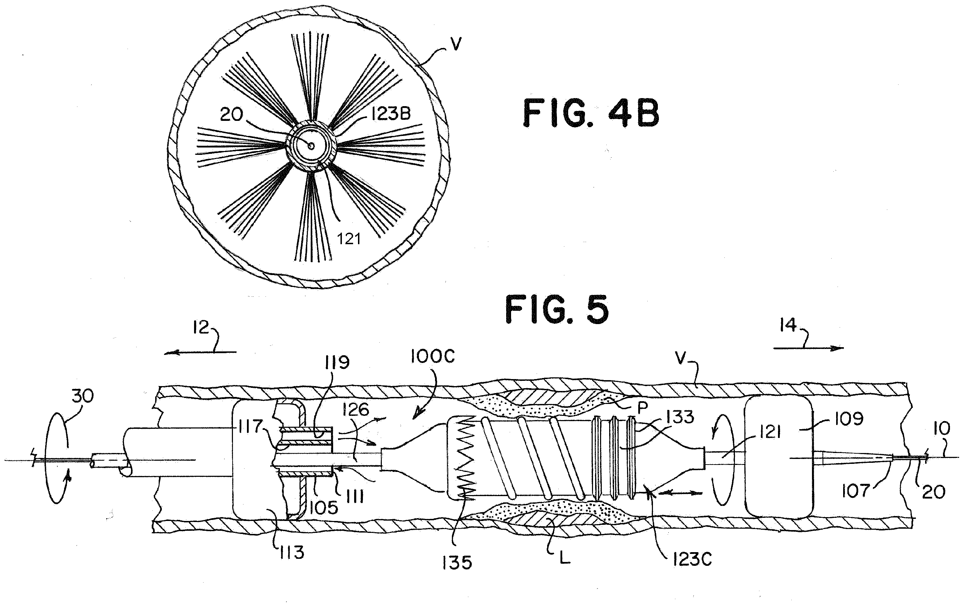

Figures 4A and 4B shows another example intravascular system 100B that can be used to displace, flush, and/or aspirate plaque. Figure 4B is a cross-sectional view of the system 100B as indicated in Figure 4A. The system 100B can include a balloon guide catheter such as the BGC 105 illustrated in the previous figures. The system 100B can include an inner tube 121 such the inner tube 121 illustrated in Figures 1 and 2A through 2I. Alternatively, the inner tube 121 can include an incision tool 123A and inner tube 121A as illustrated in Figures 3A through 3E. The system 100B can include agitation veins 123B that, when placed against the plaque, can cause portions of the plaque to become displaced. The agitation veins 123B can be collapsed within the device delivery lumen 117 during delivery of the system 100 to the lesion L. The agitation veins 123B can extend radially such that the veins 123B encompass the width of the cavity upon movement of the veins 123B out of the device delivery lumen 117. The system 100B can include a delivery tube 126 similar to the delivery tube 126 illustrated in Figures 1 and 2A through 2I. The veins can be affixed directly to the delivery tube 126. The delivery tube 126 can be manipulated to move the plaque displacement apparatus 123b distally and proximally through the cavity C. In some examples, the delivery tube 126 can be rotatable in the rotational direction 30 to rotate the plaque displacement apparatus 123B. The cavity C can be aspirated while the displacement apparatus 123B is moved to displace plaque P as described elsewhere herein.

Figure 5 shows another example intravascular system 100C that can be used to displace, flush, and/or aspirate plaque. The system 100C can include a balloon guide catheter such as the BGC 105 illustrated in the previous figures. The system 100C can include an inner tube 121 such the inner tube 121 illustrated in Figures 1 and 2A through 2I. Alternatively, the inner tube 121 can include an incision tool 123A and inner tube 121A as illustrated in Figures 3A through 3E. The system 100C can include a plaque displacement tool 123C can be inflatable and can have ridges 133 and/or barbs 135 that are positioned to expand into the plaque P when the displacement tool 123C is inflated. The displacement tool 123C can be deflated and collapsed within the device delivery lumen 117 when the system 100C is delivered to the treatment site. When the ridges 133 or barbs 135 are placed against the plaque, this can cause portions of the plaque to become displaced. The system 100C can include a delivery tube 126 similar to the delivery tube 126 illustrated in Figures 1 and 2A through 2I. The veins can be affixed directly to the delivery tube 126. The delivery tube 126 can be manipulated to move the plaque displacement apparatus 123C distally and proximally through the cavity C. In some examples, the delivery tube 126 can be rotatable in the rotational direction 30 to rotate the plaque displacement apparatus 123C. The cavity C can be aspirated while the displacement apparatus 123C is moved to displace plaque P as described elsewhere herein.

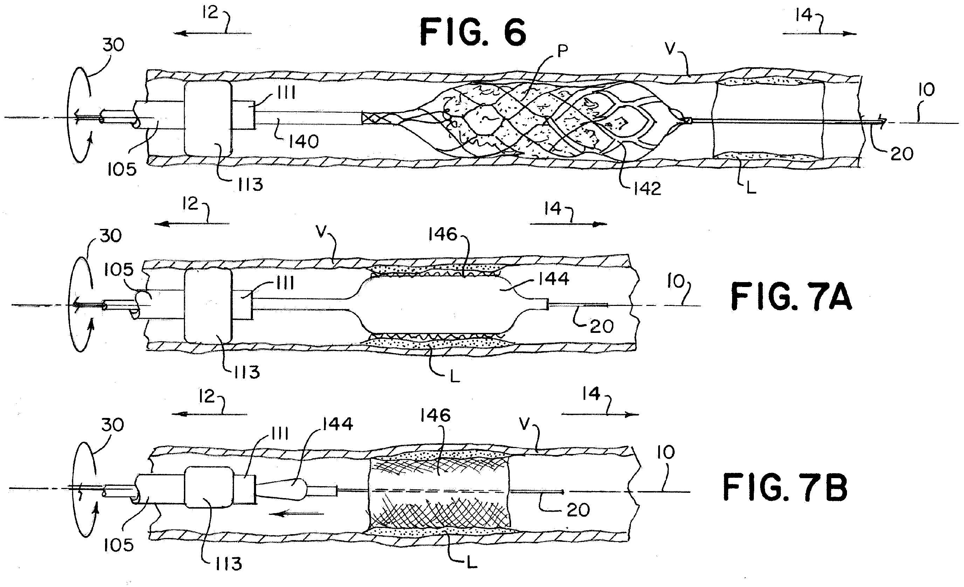

Figure 6 is an illustration of a thrombectomy device 142 including an expandable frame which can engage and pull a clot. Methods for treatment using any of the example systems 100, 100A, 100B, 100C can further include a step whereby the thrombectomy device 142 is used to extract clot material or plaque P. The thrombectomy device 142 can be delivered within a microcatheter 140 through the device delivery lumen 117 or another lumen of the BGC 105 as illustrated. Additionally, the thrombectomy device can be delivered through a lumen of the inner tube 121, 121A of any of the example systems 100, 100A, 100B, 100C. Delivery of the thrombectomy device 142 through the inner tube 121, 121A does not preclude performance of the thrombectomy as illustrated in Figure 6.

The thrombectomy can be perform by methods known to a person of ordinary skill in the art. Generally, a thrombectomy can be performed as follows. The microcatheter 140 with the thrombectomy device 142 therein can be placed across a clot, likely crossing the lesion L in the process. The microcatheter 140 can be retracted in the proximal direction 12 to deploy the thrombectomy device 142. The expanded thrombectomy device 142 with clot material therein can be extracted from the vessel V.

A thrombectomy can be performed before, after, and/or between other treatment steps illustrated herein as apparent to a person skilled in the pertinent art. In some treatments, a clot may become lodged in the distal direction 14 in relation to the lesion L. In such cases, when the inner tube 121, 121A includes a lumen sized to allow the microcatheter 140 and thrombectomy device to traverse therethrough, the lesion L can be isolated by expanded proximal and distal occlusion elements, and the lesion L can remain isolated while the thrombectomy device 142 is delivered out the distal end of the inner tube 121 to the clot.

Figures 7A and 7B are illustrations of a stent 146 being expanded by a balloon 144 into the lesion L. Methods for treatment using any of the example systems 100, 100A, 100B, 100C can further include a step whereby the stent 146 is implanted into the lesion L. The stent 146 and balloon 144 can be delivered within a microcatheter 400 through the device delivery lumen 117 or another lumen of the BGC 105.

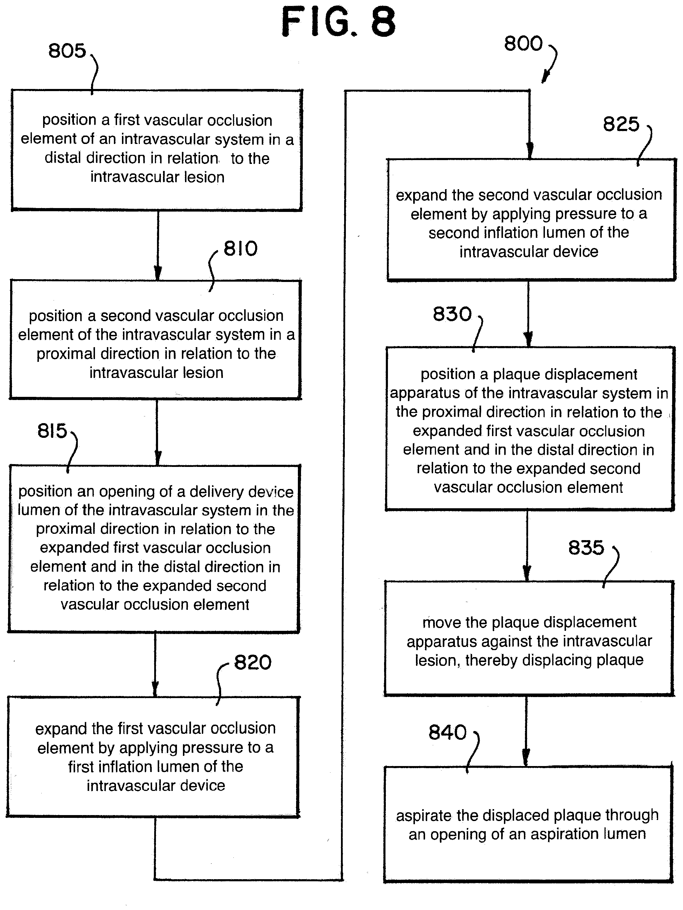

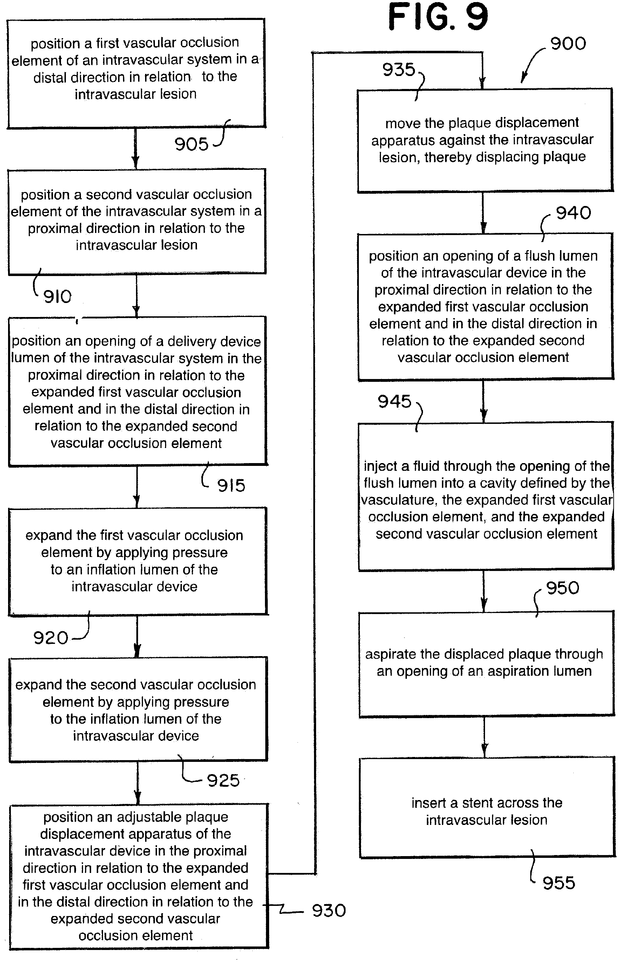

Figures 8 and 9 respectively depict example flowcharts of methods 800, 900 for displacing intravascular plaque. Each method 800, 900 can be performed, for example, by a healthcare professional using any of the intravascular systems 100, 100A-C, as disclosed herein, a variation thereof, or an alternative thereto as would be appreciated and understood by a person of ordinary skill in the art. Each method 800, 900 can respectively include one or more of the following steps presented in no particular order. Each method 800, 900 can include additional steps as would be appreciated and understood by a person of ordinary skill in the art.

Referring to the method 800 illustrated in Figure 8, at step 805, a first vascular occlusion element (e.g., the distal occlusion element 109) of an intravascular system can be positioned in a distal direction in relation to an intravascular lesion. At step 810, a second vascular occlusion element (e.g., the proximal balloon 113) of the intravascular system can be positioned in a proximal direction in relation to the intravascular lesion. At step 815, an opening of the device delivery lumen of the intravascular system can be positioned in the proximal direction in relation to the first vascular occlusion element and in the distal direction in relation to the expanded second vascular occlusion element. Once the intravascular system is properly inserted in the intravascular lesion, steps 820 and 825 can be used to expand the first vascular occlusion element and the second vascular occlusion element, respectively. The first and second vascular occlusion element can each respectively be self-expandable, mechanical expandable, inflatable, or otherwise expandable by methods known to a person of ordinary skill in the art according to the teachings herein. In some examples, the first and/or second occlusion element can be expanded by applying pressure to the inflation lumen of the intravascular system. can also serve as an anchoring point to support distal advancement of the collapsed distal expanding element.

After the first and second vascular occlusion elements are expanded, blood flow in the intravascular lesion can be restricted. Plaque within the vasculature to be displaced and removed. At step 830, a plaque displacement apparatus can be positioned in the proximal direction in relation to the expanded first vascular occlusion element and in the distal direction in relation to the expanded second vascular occlusion element. Then, at step 835, the plaque displacement apparatus can be moved against the intravascular lesion causing plaque to be displaced. At step 840, the displaced plaque can then be aspirated. In some examples, the displaced plaque can be aspirated through the opening of the device delivery lumen.

Figure 9 illustrates an example flowchart of method 900 for displacing, aspirating, and flushing intravascular plaque. At step 905, the first vascular occlusion element of the intravascular system can be positioned in the distal direction in relation to the intravascular lesion. At step 910, the second vascular occlusion element of the intravascular system can be positioned in the proximal direction in relation to the intravascular lesion. Then, at step 915, an opening of a device delivery lumen of the intravascular system can be positioned in the proximal direction in relation to the first vascular occlusion element and in the distal direction in relation to the second vascular occlusion element. Steps 920 and 925 can be performed to restrict blood flow in the vasculature (e.g., the area near the intravascular lesion). At step 920, the first vascular occlusion element can be expanded. At step 925, the second vascular occlusion element can be expanded. The first and second vascular occlusion element can each respectively be self-expandable, mechanical expandable, inflatable, or otherwise expandable by methods known to a person of ordinary skill in the art according to the teachings herein. In some examples, the first and/or second occlusion element can be expanded by applying pressure to the inflation lumen of the intravascular system.

At step 930, a plaque displacement apparatus of the intravascular system can be positioned in the proximal direction in relation to the expanded first vascular occlusion element and in the distal direction in relation to the expanded second vascular occlusion element. At step 935, the plaque displacement apparatus can be moved against the intravascular lesion to displace plaque. At step 940, an opening of the flush lumen of the intravascular system can be positioned in the proximal direction in relation to the expanded first vascular occlusion element and in the distal direction in relation to the expanded second vascular occlusion element. At step 945, a cavity of the vasculature can be flushed by injecting fluid (e.g., saline, an anti-thrombogenic drug, or a plaque sealant) through the opening of the flush lumen and into the cavity. At step 950, the displaced plaque and the fluid can be aspirated. In some examples the displaced plaque can be aspirated through an opening of a device delivery lumen. Further, at step 955, a stent can be implanted across the intravascular lesion.

The descriptions contained herein are examples of embodiments of the invention and are not intended in any way to limit the scope of the invention. As described herein, the invention contemplates many variations and modifications of the intravascular treatment system, including alternative materials, alternative device structures, alternative treatment steps, etc. Modifications apparent to those having ordinary skill in the art to which this invention relates The scope of the invention is therefore defined by the claims.

## Claims

1. A system for extracting plaque from a vasculature, the system comprising:
a balloon guide catheter (105) comprising a balloon guide catheter distal end (111), an expandable proximal balloon (113) positioned approximate the balloon guide catheter distal end, and a device delivery lumen (117) extending through the balloon guide catheter;
an inner tube (121A) extending through the device delivery lumen, the inner tube comprising a tube distal end (107) and an expandable distal occlusion element (109) positioned approximate the tube distal end and movable to exit the device delivery lumen from the balloon guide catheter distal end; and
an adjustable plaque displacement apparatus (123A) positioned within the device delivery lumen, the adjustable plaque displacement apparatus being movable in at least one of a longitudinal and rotational direction in relation to the balloon guide catheter; and
**characterised in that** the inner tube (121A) comprises an opening (131), and wherein the adjustable plaque displacement apparatus comprises an incision tool (123A) positioned within the inner tube (121A) and configured to be movable to exit the opening (131) in the inner tube (121A).

2. The system of Claim 1, wherein the balloon guide catheter comprises an aspiration lumen.

3. The system of Claim 1, wherein the plaque displacement apparatus comprises a needle.

4. The system of Claim 1, wherein the balloon guide catheter further comprises a flush lumen extending through the balloon guide catheter and comprising a third opening positioned distal to the proximal balloon.

5. The system of Claim 4, wherein the flush lumen is configured to deliver at least one of: saline, an anti-thrombogenic drug, and a plaque sealant.

## Patentansprüche

1. System zum Extrahieren von Plaque aus einer Vaskulatur, wobei das System Folgendes umfasst:
einen Ballonführungskatheter (105), der ein distales Ballonführungskatheterende (111), einen expandierbaren proximalen Ballon (113), der in der Nähe des distalen Ballonführungskatheterendes positioniert ist, und ein Vorrichtungszuführungslumen (117) umfasst, das sich durch den Ballonführungskatheter erstreckt,
eine innere Röhre (121A), die sich durch das Vorrichtungszuführungslumen erstreckt, wobei die innere Röhre ein distales Röhrenende (107) und ein expandierbares distales Okklusionselement (109) umfasst, das in der Nähe des distalen Röhrenendes positioniert ist und bewegt werden kann, um von dem distalen Ende des Ballonführungskatheters aus dem Vorrichtungszuführungslumen auszutreten, und
eine verstellbare Plaque-Entfernungsvorrichtung (123A), die in dem Vorrichtungszuführungslumen positioniert ist, wobei die verstellbare Plaque-Entfernungsvorrichtung bezüglich des Ballonführungskatheters in einer Längs und/oder einer Drehrichtung beweglich ist, und
**dadurch gekennzeichnet, dass** die innere Röhre (121A) eine Öffnung (131) umfasst, und wobei die verstellbare Plaque-Entfernungsvorrichtung ein Inzisionswerkzeug (123A) umfasst, das in der inneren Röhre (121A) positioniert und so ausgestaltet ist, dass es bewegt werden kann, um aus der Öffnung (131) in der inneren Röhre (121A) auszutreten.

2. System nach Anspruch 1, wobei der Ballonführungskatheter ein Aspirationslumen umfasst.

3. System nach Anspruch 1, wobei die Plaqueablösungsvorrichtung eine Nadel umfasst.

4. System nach Anspruch 1, wobei der Ballonführungskatheter ferner ein Spüllumen umfasst, das sich durch den Ballonführungskatheter erstreckt und eine dritte Öffnung umfasst, die distal zu dem proximalen Ballon positioniert ist.

5. System nach Anspruch 4, wobei das Spüllumen dazu ausgestaltet ist, Kochsalzlösung und/oder ein antithrombogenes Medikament und/oder ein Plaque-Abdichtungsmittel zuzuführen.

## Revendications

1. Système d'extraction de plaque dans des vaisseaux, le système comprenant :
un cathéter-guide à ballonnet (105) comprenant une extrémité distale (111) de cathéter-guide à ballonnet, un ballonnet proximal dilatable (113) positionné à proximité de l'extrémité distale de cathéter-guide à ballonnet, et une lumière de pose de dispositif (117) s'étendant à travers le cathéter-guide à ballonnet ;
un tube interne (121A) s'étendant à travers la lumière de pose de dispositif, le tube interne comprenant une extrémité distale de tube (107) et un élément d'occlusion distal dilatable (109) positionné à proximité de l'extrémité distale de tube et mobile pour sortir de la lumière de pose de dispositif depuis l'extrémité distale de cathéter-guide à ballonnet ; et
un appareil de déplacement de plaque ajustable (123A) positionné à l'intérieur de la lumière de pose de dispositif, l'appareil de déplacement de plaque ajustable étant mobile dans une direction longitudinale et/ou de rotation par rapport au cathéter-guide à ballonnet ; et
**caractérisé en ce que** le tube interne (121A) comprend une ouverture (131), et l'appareil de déplacement de plaque ajustable comprenant un outil d'incision (123A) positionné à l'intérieur du tube interne (121A) et conçu pour être mobile pour sortir par l'ouverture (131) dans le tube interne (121A).

2. Système selon la revendication 1, le cathéter-guide à ballonnet comprenant une lumière d'aspiration.

3. Système selon la revendication 1, l'appareil de déplacement de plaque comprenant une aiguille.

4. Système selon la revendication 1, le cathéter-guide à ballonnet comprenant en outre une lumière de rinçage s'étendant à travers le cathéter-guide à ballonnet et comprenant une troisième ouverture positionnée distalement par rapport au ballonnet proximal.

5. Système selon la revendication 4, la lumière de rinçage étant conçue pour administrer au moins l'un parmi : une solution saline, un médicament antithrombogène et un agent de scellement de plaque.
